# EUROPEAN PATENT SPECIFICATION

(11) **EP 3 023 094 B1**
(45) Date of publication and mention of the grant of the patent: **14.07.2021**
(21) Application number: 15194943.5
(22) Date of filing: 17.11.2015
(51) Int. Cl.: A61K 31/4709, A61P 7/02, A61P 9/00, A61P 9/08, A61P 9/10, A61P 43/00, A61K 9/20

(54) **NOVEL FORMULATION OF CILOSTAZOL**
NEUARTIGE FORMULIERUNG VON CILOSTAZOL
NOUVELLE FORMULATION DE CILOSTAZOL

(30) Priority: 18.11.2014 US 201414546864
(43) Date of publication of application: 25.05.2016
(73) Proprietor: Genovate Biotechnology Co. Ltd., Taiwan (TW)
(72) Inventor: CHEN, Fengchu, Hsin-Chu, Taiwan (TW); WANG, Yinshan, Hsin-Chu, Taiwan (TW); YANG, Yilun, Zhanghua County, Taiwan (TW); LIN, Yuying, Xinzhu County, Taiwan (TW)
(74) Representative: Cabinet Becker et Associés

(56) References cited:
- EP-A1- 1 407 785
- WO-A1-2006/030301
- US-A1- 2005 255 155
- US-B1- 7 138 143

## Description

### FIELD OF THE INVENTION

The present invention relates to pharmaceutical formulations of cilostazol, a phosphodiesterase inhibitor, more particularly to extended-release dosage forms suitable for administration once daily.

### BACKGROUND OF THE INVENTION

Cilostazol, a selective inhibitor of phosphodiesterase-3, inhibits platelet aggregation and acts as a direct arterial vasodilator. It is commercially available as Pletaal® tablets manufactured by Otsuka Pharmaceutical, the listed indications of which include relieving symptoms of intermittent claudication.

Patients suffering from intermittent claudication easily develop leg pain and limp and cannot walk a long distance without taking a rest. The intensity of the disease can be clinically measured either by initial claudication distance, i.e., the distance a patient can walk before a pain develops, or by absolute claudication distance, i.e., the distance a patient can walk until a rest has to be taken.

Intermittent claudication, a common disease among the elderly, is a clinical manifestation of peripheral vascular disease, often referred to as peripheral artery occlusive disease. Its causes include atherosclerotic lesions and disorders in platelet activation, which result in gradually narrowed arteries and ischemia symptoms. Cilostazol, a phosphodiesterase-3 inhibitor, and its metabolites elevate the concentration of cAMP in blood by blocking its metabolism, leading to therapeutic effects of anti-platelet aggregation and blood vessel expansion.

Cilostazol has been used for treating intermittent claudication. 50 and 100 mg Pletaal® tablets require two administrations per day. They are immediate-release tablets that disintegrate rapidly in the body and can cause serious adverse reactions when the cilostazol concentration in blood rise abruptly. Reported side effects attributable to cilostazol include headache, abnormal stools, diarrhea, dizziness, and palpitations.

International patent application WO 2006/030301 describes a cilostazol composition comprising starch, hydroxymethylcellulose (HPMC), and magnesium stearate.

European patent application EP 1407785 teaches a cilostazol composition comprising HPMC, D-mannitol, sodium laurylsulfate and taurine.

US patent application No. 2005/0255155 discloses micronized cilostazol particles in corn starch, microcrystalline cellulose, HPMC and magnesium stearate.

US patent 7,138,143 discloses a pharmaceutical composition containing particulate cilostazol and hydroxypropyl methylcellulose acetate succinate (HPMCAS), HPMC, sodium laurylsulfate.

There is a need to develop an extended-release form of cilostazol that, upon administration, achieves a more stable cilostazol blood concentration that would contribute to fewer side effects. In addition, an extended-release form can be taken only once per day, thereby facilitating patient compliance.

### SUMMARY OF THE INVENTION

This invention provides a pharmaceutical composition, i.e., an extended-release form of cilostazol, which, unexpectedly, has a higher efficacy and fewer side effects than Pletaal®. As such, it can be administered once daily for treating intermittent claudication.

One aspect of this invention relates to a pharmaceutical composition in solid form that contains particulate cilostazol or a salt thereof, a water-soluble cellulose, and a non-water soluble cellulose, a diluent, and a lubricant, as defined in claim 1. The particulate cilostazol or salt thereof has a 90% particle size in a cumulative particle size distribution of 5-75 µm (preferably, 10-30 µm) and constitutes 15% to 70% (preferably, 25% to 55%) by weight of the pharmaceutical composition. The pharmaceutical composition of this invention preferably features an *in vivo* plasma profile for cilostazol of C₂₄ₕ/Cₘₐₓ > 0.25 (preferably, C₂₄ₕ/Cₘₐₓ > 0.5).

The non-water-soluble cellulose is ethylcellulose (EC), cellulose acetate, methylcellulose, or a combination thereof.

The water soluble cellulose can be hydroxypropylmethylcellulose (HPMC), hydroxyethyl cellulose, hydroxyethylmethyl cellulose, hydroxypropyl cellulose, or a combination thereof.

Preferably the non water-soluble cellulose is EC and the water soluble cellulose is HPMC.

On the other hand, the diluent can be calcium carbonate, calcium phosphate, calcium sulfate, dextrates, dextrose, erythritol, fructose, kaolin, lactitol, lactose, mannitol, simethicone, sodium chloride, sorbitol, starch, sucrose, sulfobutylether-β-cyclodextrin, trehalose, xylitol, microcrystalline cellulose, or a combination thereof. Lactose, microcrystalline cellulose, and a combination thereof are preferred.

Examples of the lubricant include calcium stearate, glycerin monostearate, glyceryl behenate, glyceryl palmitostearate, hydrogenated castor oil, hydrogenated vegetable oil, light mineral oil, sodium lauryl sulfate, sodium stearyl fumarate, stearic acid, zinc stearate, sodium benzoate, magnesium stearate, myristic acid, palmitic acid, poloxamer, polyethylene glycol, potassium benzoate, talc, and a combination thereof. Magnesium stearate, stearic acid, and a combination thereof are preferred.

In one embodiment, this pharmaceutical composition contains particulate cilostazol or a salt thereof in the amount of 100 mg. In another embodiment, the amount of particulate cilostazol or a salt thereof is 200 mg.

Another aspect of this invention relates to a method of preparing the above-described pharmaceutical composition. The method includes the following steps: (i) mixing particulate cilostazol or a salt thereof having a 90% particle size in a cumulative particle size distribution of 5 to 75 µm, a water soluble cellulose, a diluent, and water to form a homogenous mixture; (ii) granulating the homogenous mixture to form granules; (iii) heating the granules to form dried granules; (iv) mixing the dried granules, a lubricant, and optionally a second cellulose (i.e., different from the first cellulose) to form a blend; and (v) compressing the blend to form tablets. The pharmaceutical composition thus prepared contains particulate cilostazol or a salt thereof, a first cellulose, a diluent, a lubricant, and optionally a second cellulose.

The water soluble cellulose can be HPMC, hydroxyethyl cellulose, hydroxyethylmethyl cellulose, hydroxypropyl cellulose, or a combination thereof.

The non water soluble cellulose is EC, methylcellulose, or a combination thereof.

Examples of the diluent and the lubricant are enumerated above.

In a preferred embodiment, the diluent is calcium carbonate, calcium phosphate, calcium sulfate, dextrates, dextrose, erythritol, fructose, kaolin, lactitol, lactose, mannitol, simethicone, sodium chloride, sorbitol, starch, sucrose, sulfobutylether-β-cyclodextrin, trehalose, xylitol, microcrystalline cellulose, or a combination thereof; the lubricant is calcium stearate, glycerin monostearate, glyceryl behenate, glyceryl palmitostearate, hydrogenated castor oil, hydrogenated vegetable oil, light mineral oil, sodium lauryl sulfate, sodium stearyl fumarate, stearic acid, zinc stearate, sodium benzoate, magnesium stearate, myristic acid, palmitic acid, poloxamer, polyethylene glycol, potassium benzoate, talc, or a combination thereof; and the second cellulose is ethylcellulose, cellulose acetate phthalate, cellulose acetate, methylcellulose, hypromellose phthalate, or a combination thereof.

The details of the invention are set forth in the drawings and description below. Other features, objects, and advantages of the invention will be apparent from the description and from the claims.

### BRIEF DESCRIPTION OF THE DRAWINGS

FIG. 1 illustrates exemplary release rate profiles for four representative cilostazol compositions, i.e., Examples 1-4, in accordance with the teachings of this invention.
FIG. 2 illustrates exemplary release rate profiles for three representative cilostazol compositions, i.e., Examples 5-7, in accordance with the teachings of this invention.
FIG. 3 illustrates exemplary release rate profiles for three representative cilostazol compositions, i.e., Examples 8-10, in accordance with the teachings of this invention.
FIG. 4 illustrates exemplary release rate profiles for four representative cilostazol compositions, i.e., Examples 11-14, in accordance with the teachings of this invention.
FIG. 5 illustrates exemplary *in vivo* plasma profiles for two representative cilostazol compositions, i.e., Examples 15 and 16, in accordance with the teachings of this invention.

### DETAILED DESCRIPTION

The pharmaceutical composition of this invention can be a Pletaal® modified release tablet, which is referred herein as "PMR." PMR is an extended-release form of cilostazol. By contrast, Pletaal® is an immediate-release cilostazol tablet.

The PMR tablet contains as an active ingredient particulate cilostazol or a salt thereof. Particulation of cilostazol, a drug insoluble in water, enhances its bioavailability. The cumulative particle size distribution of the particulate cilostazol or salt thereof is measured using a Malvern Mastersizer according to the known method described in International Patent Application Publication WO 2007/027612. D(0.9) is defined as the size of 90% of the particles based on the measured cumulative particle size distribution. Likewise, D(0.5) and D(0.1) are defined as the sizes of 50% and 10% of the particles, respectively. D(0.9) of particulate cilostazol or a salt thereof is preferably 10-20 µm and, more preferably, 10-15 µm; D(0.5) is preferably 5-10 µm and, more preferably, 6-8 µm; and D(0.1) is preferably 0.3-1 µm and, more preferably, 0.4-0.7 µm.

This particulate cilostazol or salt thereof constitutes 15% to 70% by weight of the pharmaceutical composition. To maintain the therapeutic effect, a PMR tablet has to contain the active ingredient above a minimal level (e.g., 15 wt%). On the other hand, excessive levels of the active ingredient (e.g., > 70 wt%) undermine the extended-release capability of a PMR tablet.

The PMR tablet also contains one or more celluloses, as defined by the claims, which are water-soluble or non-water soluble.

Water-soluble cellulose, when dissolved in water, forms porous hydrophilic colloid matrices so that a slow release of the drug trapped in the colloid matrices is achieved. Examples of this type of cellulose include HPMC, hydroxyethyl cellulose, hydroxyethylmethyl cellulose, and hydroxypropyl cellulose. HPMC having a high viscosity is preferred. For example, at 25 °C, a 2% (w/v) HPMC in an aqueous solution can have a viscosity of > 10,000 mPa, even > 50,000 mPa. The water-soluble cellulose constitutes 1% to 25% (preferably, 2% to 10%) by weight of the pharmaceutical composition.

Non-water-soluble cellulose acts to adjust the dissolution rate of the drug in a pharmaceutical composition. Examples include EC, cellulose acetate and methylcellulose. EC having a medium viscosity is preferred. For example, at 25 °C, a 5% (w/v) EC in a toluene/ethanol (80:20 w/w) solution can have a viscosity of 12-110 mPa, 18-80 mPa being preferred and 40-52 mPa being more preferred. The non-water-soluble cellulose, if used, constitutes no more than 50% (preferably, 20% to 40%) by weight of the pharmaceutical composition.

The PMR tablet can include, among others, Povidone, that acts as a binder to adjust the dissolution rate of the active ingredient. When Povidone is used, the Povidone products having a molecular weight of 8,000-400,000 are preferred and those having a molecular weight of 30,000-100,000 are more preferred. The diluent constitutes no more than 25% (preferably, 2% to 10%) by weight of the pharmaceutical composition.

In *in vitro* dissolution tests, the PMR tablet exhibited a profile of zero degree release. In pharmacokinetics (PK) studies after a single dose in healthy adult human subjects, this tablet exhibited an *in vivo* plasma profile superior to that of Pletaal®. More specifically, the PMR tablet showed a maximum plasma concentration (Cₘₐₓ) lower than that of Pletaal® and a plasma concentration at the 24th hour (C₂₄ₕ) greater than that of Pletaal®, when these two tablets were administered each with an equivalent amount of cilostazol. Furthermore, the PMR tablet exhibited a ratio of C₂₄ₕ to Cₘₐₓ > 0.25.

In the above-mentioned PK studies, the PMR tablet exhibited a bioavailability, as expressed conventionally by the area under curve (AUC) of 4600-13400 ng*hr/ml. Moreover, it exhibited a Cmax of 280-800 ng/ml and a C₂₄ₕ of > 0.1 µg/ml.

In one embodiment, the PMR tablet has particulate cilostazol or a salt thereof in the amount of 100 mg and features a Cmax of 280-660 ng/ml and an AUC of 4600- 7900 ng*hr/ml.

In another embodiment, the PMR tablet has particulate cilostazol or a salt thereof in the amount of 200 mg and features a Cmax of 470-800 ng/ml and an AUC of 6400-13400 ng*hr/ml.

Described below are exemplary procedures for preparing PMR tablets of this invention.

Table 1 lists four different workflow models based on which a different combination of a cellulose and a diluent are used to prepare a PMR tablet. Take Model 1 for example. First, particulate cilostazol or a salt thereof having a D(0.9) of 5-75 µm, a diluent (not shown in Table 1), e.g., lactose, and HPMC are mixed with water to form a homogenous mixture. Next, the homogenous mixture is granulated to form granules, followed by heat-drying. The dried granules are then mixed with a lubricant (not shown in Table 1), e.g., stearic acid, to form a blend. Finally, the blend is compressed to form tablets.

**Table 1. Four models for preparing a PMR**

| | Model 1 | Model 2 | Model 3 | Model 4 |
|---|---|---|---|---|
| Before granulation | A | A, B | A | A, C |
| After granulation | - | - | C | C |

| | | | | |
|---|---|---|---|---|
| A: a water-soluble cellulose B: a binder (e.g., Povidone) C: a non-water-soluble cellulose | | | | |

The specific examples below are to be construed as merely illustrative, and not limitative of the remainder of the disclosure in any way whatsoever.

Examples 1-10, not comprising water-insoluble celluloses are considered as compositions not according to the invention.

Also ex. 11-12, relating to ethyl cellulose in a weight % outside the scope of claim 1 is considered as not according to the invention.

### EXAMPLES 1-4: Extended-release tablets of cilostazol and their in vitro dissolution profiles

PMR Examples 1-4, each containing non-particulate cilostazol in the amount of 100 mg, were prepared from the ingredients shown in Table 2 below following workflow Model 1 described in Table 1.

**Table 2. Compositions for PMR Examples 1-4.**

| Ingredients | Example 1 (mg) | Example 2 (mg) | Example 3 (mg) | Example 4 (mg) |
|---|---|---|---|---|
| Cilostazol | 100 | 100 | 100 | 100 |
| Lactose anhydrous | 80 | 53 | 73 | 63 |
| HPMC K100M | 13 | 40 | 20 | 30 |
| Stearic acid | 7 | 7 | 7 | 7 |
| Total | 200 | 200 | 200 | 200 |

A study was conducted to assess the *in vitro* dissolution profiles of Examples 1-4.

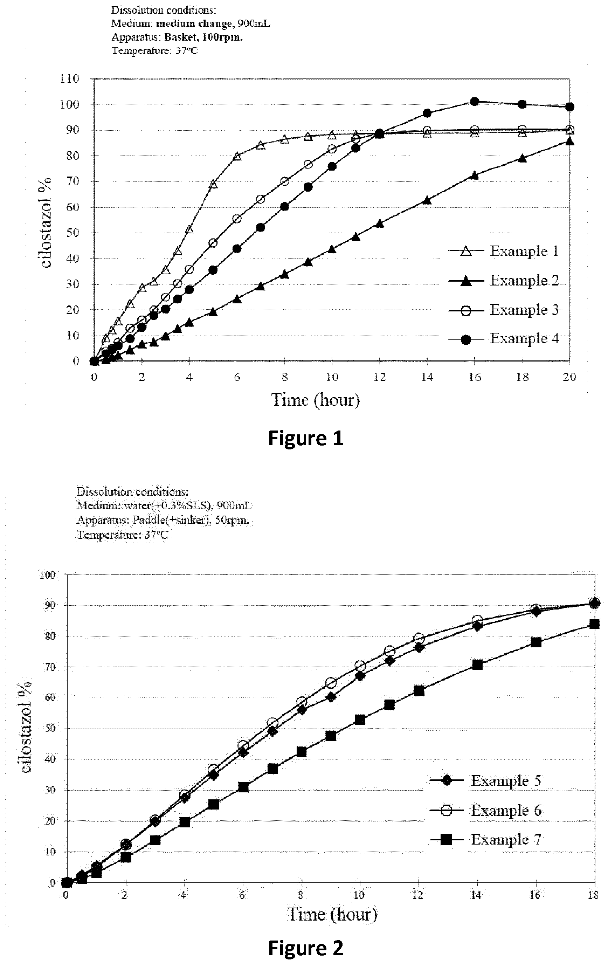

The study was performed according to the procedure described in United States Pharmacopeia (USP36, 2031). More specifically, Examples 1-4 were each placed in a dissolution medium under the temperature of about 37 °C and the dissolution medium was paddled at a speed of about 50 or 100 rpm. Cilostazol concentrations in the dissolution medium were measured at different time intervals. Results are shown in Table 3 below and Fig. 1.

**Table 3. In vitro dissolution profiles of PMR Examples 1-4.**

| Time (hour) | % released Cilostazol | | | |
|---|---|---|---|---|
| | Example 1 | Example 2 | Example 3 | Example 4 |
| 0 | 0 | 0 | 0 | 0 |
| 0.5 | 9.16 | 0.67 | 3.92 | 2.93 |
| 0.75 | 12.25 | 1.48 | 4.97 | 4.42 |
| 1.0 | 15.76 | 2.31 | 7.31 | 5.96 |
| 1.5 | 22.53 | 4.46 | 12.99 | 8.76 |
| 2.0 | 28.74 | 6.70 | 16.08 | 13.34 |
| 2.5 | 31.25 | 7.47 | 19.95 | 17.73 |
| 3 | 35.80 | 9.91 | 24.93 | 20.38 |
| 3.5 | 43.14 | 12.83 | 30.30 | 24.17 |
| 4 | 51.49 | 15.27 | 35.82 | 27.90 |
| 5 | 69.06 | 19.29 | 46.15 | 35.48 |
| 6 | 80.01 | 24.33 | 55.49 | 43.81 |
| 7 | 84.38 | 29.33 | 63.15 | 52.16 |
| 8 | 86.48 | 33.97 | 69.97 | 60.20 |
| 9 | 87.65 | 38.71 | 76.64 | 67.93 |
| 10 | 88.20 | 43.71 | 82.69 | 75.91 |
| 11 | 88.44 | 48.73 | 86.48 | 83.03 |
| 12 | 88.60 | 53.71 | 88.70 | 88.74 |
| 14 | 88.78 | 62.85 | 89.79 | 96.55 |
| 16 | 88.90 | 72.49 | 90.13 | 101.16 |
| 18 | 89.06 | 79.20 | 90.29 | 100.05 |
| 20 | 89.93 | 85.82 | 90.25 | 99.07 |

The results show that Examples 1-4 each exhibited a profile of zero degree release in a dissolution medium.

### EXAMPLES 5-7: Extended-release tablets of cilostazol and their in vitro dissolution profiles

PMR Examples 5-7, each containing particulate cilostazol in the amount of 100 mg, were prepared from the ingredients shown in Table 4 below following workflow Model 1 described in Table 1. Note that, compared to Examples 1-4, Examples 5-7 used particulate cilostazol that has a D(0.9) of 5.1-75.2 µm.

**Table 4. Compositions for PMR Examples 5-7.**

| Ingredients | Example 5 (mg) | Example 6 (mg) | Example 7 (mg) |
|---|---|---|---|
| Cilostazol | 100 D(0.9) 5.1 µm | 100 D(0.9) 13.5 µm | 100 D(0.9) 75.2 µm |
| Lactose anhydrous | 53 | 53 | 53 |
| HPMC K100M | 40 | 40 | 40 |
| Stearic acid | 7 | 7 | 7 |
| Total | 200 | 200 | 200 |

A study was conducted to assess the *in vitro* dissolution profiles of Examples 5-7. The study was performed according to the procedure described above. Results are shown in Table 5 below and Fig. 2.

**Table 5. In vitro dissolution profiles of PMR Examples 5-7.**

| Time (hour) | % released Cilostazol | | |
|---|---|---|---|
| | Example 5 | Example 6 | Example 7 |
| 0 | 0.00 | 0.00 | 0.00 |
| 0.5 | 2.54 | 2.07 | 1.33 |
| 1 | 5.57 | 5.13 | 3.28 |
| 2 | 12.33 | 12.37 | 8.23 |
| 3 | 19.92 | 20.32 | 13.83 |
| 4 | 27.52 | 28.49 | 19.66 |
| 5 | 35.00 | 36.62 | 25.34 |
| 6 | 42.21 | 44.43 | 31.04 |
| 7 | 49.12 | 51.80 | 36.97 |
| 8 | 56.08 | 58.61 | 42.51 |
| 9 | 60.18 | 64.77 | 47.75 |
| 10 | 67.10 | 70.32 | 52.83 |
| 11 | 72.09 | 75.22 | 57.70 |
| 12 | 76.43 | 79.26 | 62.35 |
| 14 | 83.28 | 85.09 | 70.73 |
| 16 | 88.07 | 88.74 | 78.01 |
| 18 | 90.68 | 90.73 | 83.98 |

The results show that, compared to Examples 1-4, Examples 5-7, which were prepared with particulate cilostazol, exhibited a more consistent profile of zero degree release in a dissolution medium. See Figs 1 and 2.

### EXAMPLES 8-10: Extended-release tablets of cilostazol and their in vitro dissolution profiles

PMR Examples 8-10, each containing particulate cilostazol in the amount of 100 mg, were prepared from the ingredients shown in Table 6 below following workflow Model 2 described in Table 1.

**Table 6. Compositions for PMR Examples 8-10.**

| Ingredients | Example 8 (mg) | Example 9 (mg) | Example 10 (mg) |
|---|---|---|---|
| Cilostazol D(0.9) < 20 µm | 100 | 100 | 100 |
| Lactose anhydrous | 53 | 53 | 53 |
| HPMC K100M | 40 | 40 | 40 |
| Stearic acid | 7 | 7 | 7 |
| Povidone K30 | 50 | 25 | 10 |
| Total | 250 | 225 | 210 |

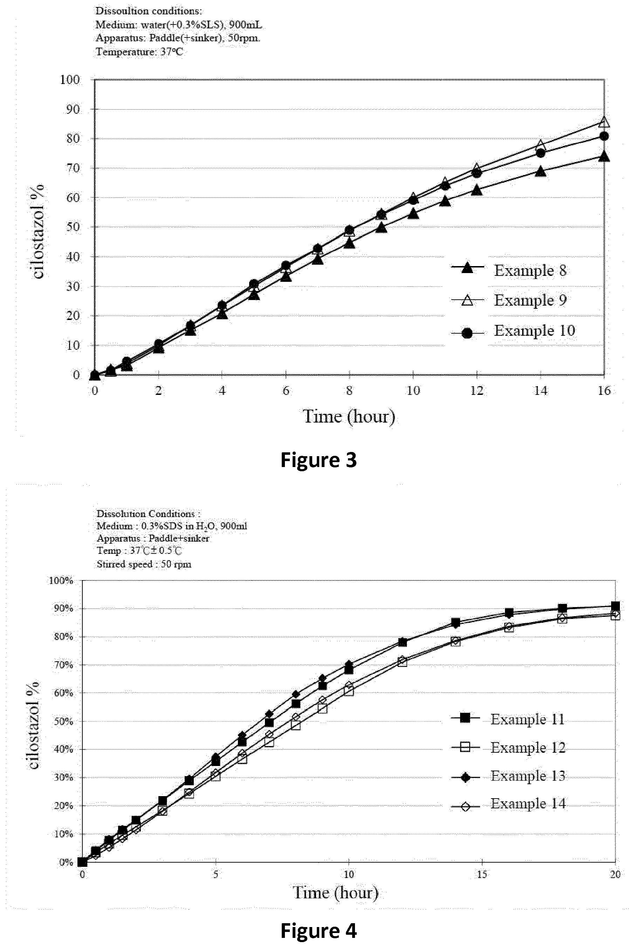

A study was conducted to assess the *in vitro* dissolution profiles of Examples 8-10. The study was performed according to the procedure described above. Results are shown in Table 7 below and Fig. 3.

**Table 7. In vitro dissolution profiles of PMR Examples 8-10.**

| Time (hour) | % released Cilostazol | | |
|---|---|---|---|
| | Example 8 | Example 9 | Example 10 |
| 0 | 0 | 0 | 0 |
| 0.5 | 1.75 | 1.42 | 1.49 |
| 1 | 3.13 | 3.92 | 4.64 |
| 2 | 9.26 | 10.00 | 10.52 |
| 3 | 15.20 | 16.78 | 16.74 |
| 4 | 20.87 | 23.52 | 23.56 |
| 5 | 27.36 | 30.12 | 30.85 |
| 6 | 33.53 | 36.60 | 37.12 |
| 7 | 39.38 | 42.75 | 42.83 |
| 8 | 44.83 | 48.78 | 48.95 |
| 9 | 50.01 | 54.48 | 54.26 |
| 10 | 54.72 | 60.02 | 59.10 |
| 11 | 58.95 | 65.15 | 63.97 |
| 12 | 62.72 | 69.92 | 68.13 |
| 14 | 69.01 | 77.87 | 75.08 |
| 16 | 74.13 | 85.75 | 80.90 |

The results show that varying amounts of Povidone products used in preparation does not impact the in vitro dissolution profile of a PMR tablet.

### EXAMPLES 11-14: Extended-release tablets of cilostazol and their in vitro dissolution profiles

PMR Examples 11-14, each containing particulate cilostazol in the amount of 200 mg, were prepared from the ingredients shown in Table 8 below following workflow Model 3 described in Table 1. Note that different compressing force was applied when tableting Examples 11-14.

**Table 8. Compositions for PMR Examples 11-14.**

| Ingredients | Example 11 (mg) | Example 12 (mg) | Example 13 (mg) | Example 14 (mg) |
|---|---|---|---|---|
| Tableting Compressing Force | 18 kg/cm² | 21 kg/cm² | 15.5 kg/c ² | 25 kg/cm² |
| Cilostazol D(0.9) < 20µm | 200 | 200 | 200 | 200 |
| Lactose anhydrous | 160 | 160 | 160 | 160 |
| HPMC K100M | 30 | 30 | 30 | 30 |
| Ethyl cellulose | 330 | 330 | 198 | 198 |
| Stearic acid | 14 | 14 | 14 | 14 |
| Total | 734 | 734 | 602 | 602 |

A study was conducted to assess the *in vitro* dissolution profiles of Examples 11-14. The study was performed according to the procedure described above. Results are shown in Table 9 below and Fig. 4.

**Table 9. In vitro dissolution profiles of PMR Examples 11-14.**

| Time (hour) | % released Cilostazol | | | |
|---|---|---|---|---|
| | Example 11 (18 kg/cm²) | Example 12 (21 kg/cm²) | Example 13 (15.5 kg/cm²) | Example 14 (25 kg/cm²) |
| 0 | 0 | 0 | 0 | 0 |
| 0.5 | 4.1 | 3.5 | 4.3 | 2.3 |
| 1 | 7.8 | 6.5 | 8.1 | 5.2 |
| 1.5 | 11.4 | 9.6 | 11.6 | 8.3 |
| 2 | 14.9 | 12.5 | 15.0 | 11.4 |
| 3 | 21.8 | 18.3 | 22.0 | 18.0 |
| 4 | 28.9 | 24.3 | 29.5 | 24.9 |
| 5 | 35.7 | 30.4 | 37.4 | 31.7 |
| 6 | 42.6 | 36.6 | 45.1 | 38.7 |
| 7 | 49.5 | 42.6 | 52.6 | 45.4 |
| 8 | 56.1 | 48.5 | 59.5 | 51.6 |
| 9 | 62.5 | 54.5 | 65.3 | 57.5 |
| 10 | 68.2 | 60.6 | 70.3 | 62.9 |
| 12 | 78.0 | 71.0 | 78.5 | 71.9 |
| 14 | 85.2 | 78.3 | 84.4 | 78.7 |
| 16 | 88.6 | 83.3 | 87.9 | 83.8 |
| 18 | 90.1 | 86.4 | 89.8 | 86.7 |
| 20 | 91.0 | 87.6 | 90.9 | 88.3 |

The results show that using a high amount of ethylcellulose, as in Examples 11 and 12, and applying compressing force during tableting, as in Examples 12 and 14, impacted the in vitro dissolution profile of a PMR tablet.

### EXAMPLES 15 AND 16: Extended-release tablets of cilostazol and their in vivo plasma profiles

PMR Examples 15 and 16, containing particulate cilostazol in the amount of 100 mg and 200 mg, respectively, were prepared from the ingredients shown in Table 10 below following workflow Model 3 described in Table 1.

**Table 10. Compositions for PMR Examples 15 and 16**

| Ingredients | Example 15 (mg) | Example 16 (mg) |
|---|---|---|
| Cilostazol D(0.9) < 20µm | 100 | 200 |
| Lactose anhydrous | 80 | 160 |
| HPMC K100M | 15 | 30 |
| Ethyl cellulose | 132 | 264 |
| Stearic acid | 7 | 14 |
| Total | 334 | 668 |

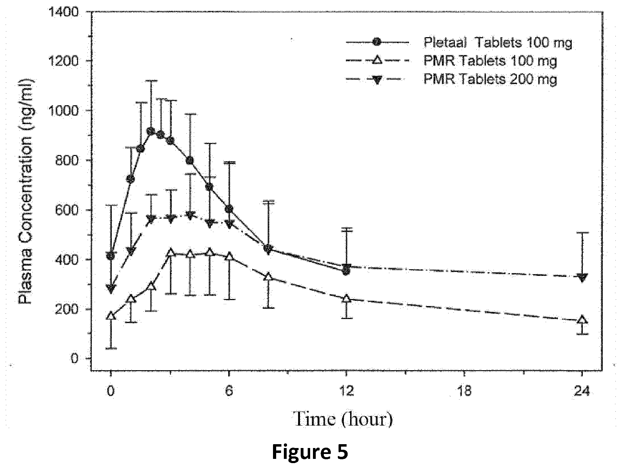

A PK study was conducted, in healthy adult human subjects after a single dose of PMR Example 15 or 16, to assess the *in vivo* plasma profiles. In addition, Pletaal, an immediate-release tablet, was used as a control in this study. Pletaal in the amount of 100 mg, Example 15, and Example 16 are designated as "Pletaal 100 mg," "PMR 100 mg," and "PMR 200 mg," respectively. Results are shown in Table 11 below and Fig. 5.

**Table 11. In vivo plasma profiles of PMR Examples 15 and 16, and a Pletaal tablet, at 0-24 hours after a single dose administration**

| Time (hour) | Pletaal 100 mg (ng/mL) | Example 15 PMR 100 mg (ng/mL) | Example 16 PMR 200 mg (ng/mL) |
|---|---|---|---|
| 0 | 412 | 169 | 287 |
| 1.0 | 722 | 239 | 438 |
| 1.5 | 844 | - | - |
| 2.0 | 914 | 289 | 566 |
| 2.5 | 899 | - | - |
| 3.0 | 877 | 425 | 568 |
| 4.0 | 796 | 420 | 581 |
| 5.0 | 690 | 427 | 550 |
| 6.0 | 601 | 409 | 547 |
| 8.0 | 444 | 327 | 442 |
| 12 | 350 | 239 | 371 |
| 24 | - | 152 | 332 |

From the same PK study, Cₘₐₓ, Tₘₐₓ (time required to reach the maximum plasma concentration), and AUC of these three samples are shown in Table 12 below.

**Table 12. Results of a PK study on PMR Examples 15 and 16, and a Pletaal tablet**

| | Cₘₐₓ (ng/ml) | Tₘₐₓ (hr) | AUC (ng^{∗}hr/ml) |
|---|---|---|---|
| Pletaal 100 mg | 982±188 | 2.4±1.0 | 7114±1712 |
| PMR 100 mg (Example 15) | 468±189 | 4.5±1.4 | 6236±1640 |
| PMR 200 mg (Example 16) | 636±163 | 3.3±2.1 | 9885±3489 |

The results demonstrate that "PMR 100 mg" (Examples 15) and "PMR 200 mg" (Examples 16) each exhibited an *in vivo* plasma profile superior to that of "Pletaal 100 mg." Namely, "PMR 100 mg" and "PMR 200 mg" each showed a Cₘₐₓ much lower than that of "Pletaal 100 mg" (468±189 and 636±163 vs. 982±188) and a Tₘₐₓ much higher than that of "Pletaal 100 mg" (4.5±1.4 and 3.3±2.1 vs. 2.4±1.0).

### EXAMPLE 17: An extended-release tablet of cilostazol and its efficacy study

PMR Example 17, containing particulate cilostazol in the amount of 200 mg, was prepared from the ingredients shown in Table 13 below following workflow Model 4 described in Table 1.

**Table 13. Compositions for PMR Example 17**

| Ingredients | Example 17 (mg) |
|---|---|
| Cilostazol D(0.9) < 20µm | 200 |
| Lactose anhydrous | 160 |
| HPMC K100M | 30 |
| Ethyl cellulose | 264 |
| Stearic acid | 7 |
| Total | 661 |

In a randomized, double-blind clinical trial, Example 17 tablets (PMR 200 mg, once daily) and Pletaal tablets (Pletaal 100 mg, twice daily) were each used to treat a group of 10 peripheral vascular disease patients separately for 16 weeks. At the beginning (0 week), the middle (8 weeks), and the end (16 weeks) of the trial, initial claudication distances (ICDs) were measured from these 20 patients. ICD improvements as a result of treatment were measured as "increase %," compared to ICDs at the beginning of the trial. Results, summarized in Table 14 below, compare the efficacy of "PMR 200 mg," i.e., Example 17, and "Pletaal 100 mg" in treating intermittent claudication.

**Table 14. An efficacy comparison between "PMR 200 mg" (once daily) and "Pletaal 100 mg" (twice daily)**

| Time (week) | PMR 200 mg, once daily (n=10) | | Pletaal 100 mg, twice daily (n=10) | |
|---|---|---|---|---|
| | Claudication distance (m) | increase % | Claudication distance (m) | increase % |
| 0 | 91.9 | - | 120.3 | - |
| 8 | 125.7 | 45.9% | 217.3 | 69.4% |
| 16 | 233.2 | 103.7% | 218.7 | 69.1% |

The results show that, at the end of 16-week trial, "PMR 200 mg," i.e., an extended release pharmaceutical composition of this invention, unexpectedly, yielded a much greater clinical improvement (103.7% vs. 69.1%) in treating intermittent claudication than "Pletaal 100 mg," an immediate-release tablet, even though "PMR 200 mg" was administered once daily and "Pletaal 100 mg" twice daily.

In the same clinical trial, "PMR 200 mg," unexpectedly, showed much fewer side effects than "Pletaal 100 mg." Specifically, much more patients reported no adverse event in the "PMR 200 mg" group than those in the "Pletaal 100 mg" group (20% vs. 10%); and much fewer patients reported drug-related adverse events in the "PMR 200 mg" group than those in the "Pletaal 100 mg" group (30% vs. 40%).

## Claims

1. A pharmaceutical composition comprising:
particulate cilostazol or a salt thereof having a 90% particle size in a cumulative particle size distribution of 5 to 75 µm;
a water-soluble cellulose;
a non-water-soluble cellulose;
a diluent; and
a lubricant,
wherein the water-soluble cellulose constitutes 1% to 25% by weight, the non-water-soluble cellulose constitutes 20% to 40% by weight, and the particulate cilostazol or salt thereof constitutes 15% to 70% by weight of the pharmaceutical composition that is in solid, extended-release form and features an *in vivo* plasma profile for cilostazol of C₂₄ₕ/Cₘₐₓ > 0.25; and the non-water-soluble cellulose is ethylcellulose, cellulose acetate, methylcellulose, or a combination thereof.

2. The pharmaceutical composition of claim 1, wherein the particulate cilostazol or salt thereof has a 90% particle size in a cumulative particle size distribution of 10 to 30 µm.

3. The pharmaceutical composition of claim 1 or 2, wherein the water-soluble cellulose is hydroxypropylmethylcellulose (HPMC), hydroxy ethyl cellulose, hydroxyethylmethyl cellulose, hydroxypropyl cellulose, or a combination thereof; and the non-water-soluble cellulose is ethylcellulose, cellulose acetate, or a combination thereof, preferably wherein the water-soluble cellulose is HPMC and the non-water-soluble cellulose is ethylcellulose.

4. The pharmaceutical composition of any of claims 1 to 3, wherein the diluent is calcium carbonate, calcium phosphate, calcium sulfate, dextrates, dextrose, erythritol, fructose, kaolin, lactitol, lactose, mannitol, simethicone, sodium chloride, sorbitol, starch, sucrose, sulfobutylether-β-cyclodextrin, trehalose, xylitol, microcrystalline cellulose, or a combination thereof, preferably wherein the diluent is lactose, microcrystalline cellulose, or a combination thereof.

5. The pharmaceutical composition of any of claims 1 to 4, wherein the lubricant is calcium stearate, glycerin monostearate, glyceryl behenate, glyceryl palmitostearate, hydrogenated castor oil, hydrogenated vegetable oil, light mineral oil, sodium lauryl sulfate, sodium stearyl fumarate, stearic acid, zinc stearate, sodium benzoate, magnesium stearate, myristic acid, palmitic acid, poloxamer, polyethylene glycol, potassium benzoate, talc, or a combination thereof, preferably wherein the lubricant is magnesium stearate, stearic acid, or a combination thereof.

6. The pharmaceutical composition of any of claims 1 to 5, wherein the water-soluble cellulose is HPMC and the non-water-soluble cellulose is ethylcellulose; the diluent is lactose, microcrystalline cellulose, or a combination thereof; and the lubricant is magnesium stearate, stearic acid, or a combination thereof.

7. The pharmaceutical composition of any of claims 1 to 6, wherein the amount of particulate cilostazol or salt thereof is 100 mg.

8. The pharmaceutical composition of any of claims 1 to 6, wherein the amount of particulate cilostazol or salt thereof is 200 mg.

9. A method of preparing the pharmaceutical composition of any of claims 1 to 8, the method comprising:
mixing particulate cilostazol or a salt thereof having a 90% particle size in a cumulative particle size distribution of 5 to 75 µm, the water-soluble cellulose, the diluent, and water to form a homogenous mixture;
granulating the homogenous mixture to form granules;
heating the granules to form dried granules;
mixing the dried granules, the lubricant, and a water-insoluble cellulose to form a blend; and
compressing the blend to form tablets;
wherein the water-insoluble cellulose is ethylcellulose, cellulose acetate, methylcellulose, or a combination thereof.

10. The method of claim 9, wherein the water-soluble cellulose is HPMC, hydroxyethyl cellulose, hydroxyethylmethyl cellulose, hydroxypropyl cellulose, ethylcellulose, cellulose acetate phthalate, cellulose acetate, or a combination thereof.

11. The method of claim 9 or 10, wherein the diluent is calcium carbonate, calcium phosphate, calcium sulfate, dextrates, dextrose, erythritol, fructose, kaolin, lactitol, lactose, mannitol, simethicone, sodium chloride, sorbitol, starch, sucrose, sulfobutylether-β-cyclodextrin, trehalose, xylitol, microcrystalline cellulose, or a combination thereof.

12. The method of any of claims 9 to 11, the lubricant is calcium stearate, glycerin monostearate, glyceryl behenate, glyceryl palmitostearate, hydrogenated castor oil, hydrogenated vegetable oil, light mineral oil, sodium lauryl sulfate, sodium stearyl fumarate, stearic acid, zinc stearate, sodium benzoate, magnesium stearate, myristic acid, palmitic acid, poloxamer, polyethylene glycol, potassium benzoate, talc, or a combination thereof.

13. The pharmaceutical composition of claim 1, wherein the pharmaceutical composition features the area under curve for cilostazol of 4600-13400 ng*hr/ml.

14. The pharmaceutical composition of claim 1, wherein the Cmax is 280-800 ng/ml and/or the C₂₄ₕ is > 0.1 µg/ml.

## Patentansprüche

1. Pharmazeutische Zusammensetzung, umfassend:
partikuläres Cilostazol oder ein Salz davon mit einer Teilchengröße von 90% in einer kumulativen Teilchengrößenverteilung von 5 bis 75 µm;
eine wasserlösliche Cellulose;
eine nicht wasserlösliche Cellulose;
ein Verdünnungsmittel; und
ein Schmiermittel,
wobei die wasserlösliche Cellulose 1 bis 25 Gew.-% ausmacht, die nicht wasserlösliche Cellulose 20 bis 40 Gew.-% ausmacht und das partikuläre Cilostazol oder Salz davon 15 bis 70 Gew.-% der pharmazeutischen Zusammensetzung ausmacht, die in fester Form mit verlängerter Freisetzung vorliegt und ein *In-vivo*-Plasmaprofil für Cilostazol von C₂₄ₕ/Cₘₐₓ > 0,25 aufweist; und die nicht wasserlösliche Cellulose Ethylcellulose, Celluloseacetat, Methylcellulose oder eine Kombination davon ist.

2. Pharmazeutische Zusammensetzung nach Anspruch 1, wobei das partikuläre Cilostazol oder Salz davon eine Teilchengröße von 90% in einer kumulativen Teilchengrößenverteilung von 10 bis 30 µm aufweist.

3. Pharmazeutische Zusammensetzung nach Anspruch 1 oder 2, wobei die wasserlösliche Cellulose Hydroxypropylmethylcellulose (HPMC), Hydroxyethylcellulose, Hydroxyethylmethylcellulose, Hydroxypropylcellulose oder eine Kombination davon ist; und die nicht wasserlösliche Cellulose Ethylcellulose, Celluloseacetat oder eine Kombination davon ist, vorzugsweise wobei die wasserlösliche Cellulose HPMC ist und die nicht wasserlösliche Cellulose Ethylcellulose ist.

4. Pharmazeutische Zusammensetzung nach einem der Ansprüche 1 bis 3, wobei das Verdünnungsmittel Calciumcarbonat, Calciumphosphat, Calciumsulfat, Dextrate, Dextrose, Erythrit, Fructose, Kaolin, Lactit, Lactose, Mannit, Simethicon, Natriumchlorid, Sorbit, Stärke, Saccharose, Sulfobutylether-β-Cyclodextrin, Trehalose, Xylit, mikrokristalline Cellulose oder eine Kombination davon ist, vorzugsweise wobei das Verdünnungsmittel Lactose, mikrokristalline Cellulose oder eine Kombination davon ist.

5. Pharmazeutische Zusammensetzung nach einem der Ansprüche 1 bis 4, wobei das Schmiermittel Calciumstearat, Glycerinmonostearat, Glycerylbehenat, Glycerylpalmitostearat, hydriertes Rizinusöl, hydriertes Pflanzenöl, leichtes Mineralöl, Natriumlaurylsulfat, Natriumstearylfumarat, Stearinsäure, Zinkstearat, Natriumbenzoat, Magnesiumstearat, Myristinsäure, Palmitinsäure, Poloxamer, Polyethylenglykol, Kaliumbenzoat, Talk oder eine Kombination davon ist, vorzugsweise wobei das Schmiermittel Magnesiumstearat, Stearinsäure oder eine Kombination davon ist.

6. Pharmazeutische Zusammensetzung nach einem der Ansprüche 1 bis 5, wobei die wasserlösliche Cellulose HPMC ist und die nicht wasserlösliche Cellulose Ethylcellulose ist; das Verdünnungsmittel Lactose, mikrokristalline Cellulose oder eine Kombination davon ist; und das Schmiermittel Magnesiumstearat, Stearinsäure oder eine Kombination davon ist.

7. Pharmazeutische Zusammensetzung nach einem der Ansprüche 1 bis 6, wobei die Menge an partikulärem Cilostazol oder Salz davon 100 mg beträgt.

8. Pharmazeutische Zusammensetzung nach einem der Ansprüche 1 bis 6, wobei die Menge an partikulärem Cilostazol oder Salz davon 200 mg beträgt.

9. Verfahren zur Herstellung der pharmazeutischen Zusammensetzung nach einem der Ansprüche 1 bis 8, wobei das Verfahren umfasst:
Mischen von partikulärem Cilostazol oder einem Salz davon mit einer Teilchengröße von 90% in einer kumulativen Teilchengrößenverteilung von 5 bis 75 µm, der wasserlöslichen Cellulose, dem Verdünnungsmittel und Wasser, um eine homogene Mischung zu bilden;
Granulieren der homogenen Mischung unter Bildung von Körnchen;
Erhitzen der Körnchen unter Bildung getrockneter Körnchen;
Mischen der getrockneten Körnchen, des Schmiermittels und einer wasserunlöslichen Cellulose, um eine Mischung zu bilden; und
Komprimieren der Mischung, um Tabletten zu bilden;
wobei die wasserunlösliche Cellulose Ethylcellulose, Celluloseacetat, Methylcellulose oder eine Kombination davon ist.

10. Verfahren nach Anspruch 9, wobei die wasserlösliche Cellulose HPMC, Hydroxyethylcellulose, Hydroxyethylmethylcellulose, Hydroxypropylcellulose, Ethylcellulose, Celluloseacetatphthalat, Celluloseacetat oder eine Kombination davon ist.

11. Verfahren nach Anspruch 9 oder 10, wobei das Verdünnungsmittel Calciumcarbonat, Calciumphosphat, Calciumsulfat, Dextrate, Dextrose, Erythrit, Fructose, Kaolin, Lactit, Lactose, Mannit, Simethicon, Natriumchlorid, Sorbit, Stärke, Saccharose, Sulfobutylether-β-Cyclodextrin, Trehalose, Xylit, mikrokristalline Cellulose oder eine Kombination davon ist.

12. Verfahren nach einem der Ansprüche 9 bis 11, das Schmiermittel Calciumstearat, Glycerinmonostearat, Glycerylbehenat, Glycerylpalmitostearat, hydriertes Rizinusöl, hydriertes Pflanzenöl, leichtes Mineralöl, Natriumlaurylsulfat, Natriumstearylfumarat, Stearinsäure, Zinkstearat, Natriumbenzoat, Magnesiumstearat, Myristinsäure, Palmitinsäure, Poloxamer, Polyethylenglykol, Kaliumbenzoat, Talk oder eine Kombination davon ist.

13. Pharmazeutische Zusammensetzung nach Anspruch 1, wobei die pharmazeutische Zusammensetzung die Kurvenfläche (area under curve) für Cilostazol von 4600-13400 ng*h/ml aufweist.

14. Pharmazeutische Zusammensetzung nach Anspruch 1, wobei die Cmax 280-800 ng/ml und/oder die C24h > 0,1 µg/ml beträgt.

## Revendications

1. Composition pharmaceutique comprenant :
du cilostazol particulaire ou un sel de celui-ci ayant une granulométrie à 90 % dans la distribution de granulométrie cumulative de 5 à 75 µm ;
une cellulose soluble dans l'eau ;
une cellulose insoluble dans l'eau ;
un diluant ; et
un lubrifiant,
dans laquelle la cellulose soluble dans l'eau constitue 1 % à 25 % en poids, la cellulose insoluble dans l'eau constitue 20 % à 40 % en poids, et le cilostazol particulaire ou son sel constitue 15 % à 70 % en poids de la composition pharmaceutique qui est sous une forme solide à libération prolongée, et caractérise un profil plasmatique *in vivo* pour le cilostazol de C₂₄ₕ/Cₘₐₓ > 0,25 ; et la cellulose insoluble dans l'eau est l'éthylcellulose, l'acétate de cellulose, la méthylcellulose, ou une de leurs combinaisons.

2. Composition pharmaceutique selon la revendication 1, dans laquelle le cilostazol particulaire ou son sel a une granulométrie à 90 % dans la distribution de granulométrie cumulative de 10 à 30 µm.

3. Composition pharmaceutique selon la revendication 1 ou 2, dans laquelle la cellulose soluble dans l'eau est l'hydroxypropylméthylcellulose (HPMC), l'hydroxyéthylcellulose, l'hydroxyéthylméthylcellulose, l'hydroxypropylcellulose, ou une de leurs combinaisons ; et la cellulose insoluble dans l'eau est l'éthylcellulose, l'acétate de cellulose, ou une de leurs combinaisons, de préférence dans laquelle la cellulose soluble dans l'eau est l'HPMC et la cellulose insoluble dans l'eau est l'éthylcellulose.

4. Composition pharmaceutique selon l'une quelconque des revendications 1 à 3, dans laquelle le diluant est le carbonate de calcium, le phosphate de calcium, le sulfate de calcium, les dextrates, le dextrose, l'érythritol, le fructose, le kaolin, le lactitol, le lactose, le mannitol, la siméthicone, le chlorure de sodium, le sorbitol, l'amidon, le saccharose, la sulfobutyléther-β-cyclodextrine, le tréhalose, le xylitol, la cellulose microcristalline, ou une de leurs combinaisons, de préférence dans laquelle le diluant est le lactose, la cellulose microcristalline, ou une de leurs combinaisons.

5. Composition pharmaceutique selon l'une quelconque des revendications 1 à 4, dans laquelle le lubrifiant est le stéarate de calcium, le monostéarate de glycérol, le béhénate de glycéryle, le palmitostéarate de glycéryle, l'huile de ricin hydrogénée, une huile végétale hydrogénée, une huile minérale légère, le laurylsulfate de sodium, le stéarylfumarate de sodium, l'acide stéarique, le stéarate de zinc, le benzoate de sodium, le stéarate de magnésium, l'acide myristique, l'acide palmitique, un poloxamère, le polyéthylèneglycol, le benzoate de potassium, le talc, ou une de leurs combinaisons, de préférence dans laquelle le lubrifiant est le stéarate de magnésium, l'acide stéarique, ou une de leurs combinaisons.

6. Composition pharmaceutique selon l'une quelconque des revendications 1 à 5, dans laquelle la cellulose soluble dans l'eau est l'HPMC et la cellulose insoluble dans l'eau est l'éthylcellulose ; le diluant est le lactose, la cellulose microcristalline, ou une de leurs combinaisons ; et le lubrifiant est le stéarate de magnésium, l'acide stéarique, ou une de leurs combinaisons.

7. Composition pharmaceutique selon l'une quelconque des revendications 1 à 6, dans laquelle la quantité de cilostazol particulaire ou de son sel est de 100 mg.

8. Composition pharmaceutique selon l'une quelconque des revendications 1 à 6, dans laquelle la quantité de cilostazol particulaire ou de son sel est de 200 mg.

9. Méthode de préparation de la composition pharmaceutique de l'une quelconque des revendications 1 à 8, la méthode comprenant :
le mélange de cilostazol particulaire ou d'un sel de celui-ci ayant une granulométrie à 90 % dans la distribution de granulométrie cumulative de 5 à 75 µm, de la cellulose soluble dans l'eau, du diluant, et d'eau, pour former un mélange homogène ;
la granulation du mélange homogène pour former des granules ;
le chauffage des granules pour former des granules séchés ;
le mélange des granules séchés, du lubrifiant, et d'une cellulose insoluble dans l'eau pour former un mélange combiné ; et
la compression du mélange combiné pour former des comprimés ;
dans laquelle la cellulose insoluble dans l'eau est l'éthylcellulose, l'acétate de cellulose, la méthylcellulose, ou une de leurs combinaisons.

10. Méthode selon la revendication 9, dans laquelle la cellulose soluble dans l'eau est l'HPMC, l'hydroxyéthylcellulose, l'hydroxyéthylméthylcellulose, l'hydroxypropylcellulose, l'éthylcellulose, l'acétophtalate de cellulose, l'acétate de cellulose, ou une de leurs combinaisons.

11. Méthode selon la revendication 9 ou 10, dans laquelle le diluant est le carbonate de calcium, le phosphate de calcium, le sulfate de calcium, les dextrates, le dextrose, l'érythritol, le fructose, le kaolin, le lactitol, le lactose, le mannitol, la siméthicone, le chlorure de sodium, le sorbitol, l'amidon, le saccharose, la sulfobutyléther-β-cyclodextrine, le tréhalose, le xylitol, la cellulose microcristalline, ou une de leurs combinaisons.

12. Méthode selon l'une quelconque des revendications 9 à 11, dans laquelle le lubrifiant est le stéarate de calcium, le monostéarate de glycérol, le béhénate de glycéryle, le palmitostéarate de glycéryle, l'huile de ricin hydrogénée, une huile végétale hydrogénée, une huile minérale légère, le laurylsulfate de sodium, le stéarylfumarate de sodium, l'acide stéarique, le stéarate de zinc, le benzoate de sodium, le stéarate de magnésium, l'acide myristique, l'acide palmitique, un poloxamère, le polyéthylèneglycol, le benzoate de potassium, le talc, ou une de leurs combinaisons.

13. Composition pharmaceutique selon la revendication 1, laquelle composition pharmaceutique se **caractérise par** une surface sous la courbe pour le cilostazol de 4 600 à 13 400 ng*h/ml.

14. Composition pharmaceutique selon la revendication 1, dans laquelle la valeur Cmax est de 280 à 800 ng/ml et/ou la valeur C₂₄ₕ est > 0,1 µg/ml.
